Europäisches Patentamt

European Patent Office

Office européen des brevets

(19)

(11) Publication number: **0 584 366 A1**

(12)

# EUROPEAN PATENT APPLICATION
## published in accordance with Art. 158(3) EPC

(21) Application number: **93904293.3**

(22) Date of filing: **10.02.93**

(86) International application number:
**PCT/JP93/00169**

(87) International publication number:
**WO 93/15867 (19.08.93 93/20)**

(51) Int. Cl.⁵: **B23H 7/08**

(30) Priority: **17.02.92 JP 61479/92**

(43) Date of publication of application:
**02.03.94 Bulletin 94/09**

(84) Designated Contracting States:
**AT BE CH DE DK ES FR GB GR IE IT LI LU MC NL PT SE**

(71) Applicant: **ISHIFUKUKINZOKU KOGYO KABUSHIKI KAISHA**
**20-7, Uchikanda 3-chome,**
**Chiyoda-ku**
**Tokyo 101(JP)**

(72) Inventor: **FUJIWARA, Masahiro,**
**Ishifukukinzokukogyo K. K.**
**Sokadaiichi-Kojo-nai,**
**12-30, Aoyagi 2-chome**
**Soka-shi, Saitama-ken 340(JP)**

Inventor: **ISHII, Nobuo, Ishifukukinzokukogyo K. K.**
**Sokadaiichi-Kojo-nai,**
**12-30, Aoyagi 2-chome**
**Soka-shi, Saitamma-ken 340(JP)**
Inventor: **HORIKAWA, Takashi,**
**Ishifukukinzokukogyo K. K.**
**Sokadaiichi-Kojo-nai,**
**12-30, Aoyagi 2-chome**
**Soka-shi, Saitama-ken 340(JP)**
Inventor: **KOYAMA, Kazuaki,**
**Ishifukukinzokugyo K. K.**
**Sokadaiichi-Kojo-nai,**
**12-30, Aoyagi 2-chome**
**Soka-shi, Saitama-ken 340(JP)**

(74) Representative: **Graf, Walter, Dipl.-Ing.**
**Sckellstrasse 1**
**D-81667 München (DE)**

(54) **METHOD OF PROCESSING METAL MATERIAL FOR MEDICAL INSTRUMENTS.**

(57) To form to a complicated shape as designed a metal material for medical instruments, such as titanium and a titanium alloy, which have a high non-eluting feature and a high corrosion resistance as materials to he implanted and as materials for medical instruments, such as an instrument for a surgical operation, by excellent precision processing techniques, i.e. electric discharge machining, whereby a product can fully display the function thereof specific to a medical instrument, and be furnished with a high non-eluting feature and a high corrosion resistance. When the electric discharge machining having an excellent processability is applied to the production of a medical instrument from a metal material for the production of such instruments which has a high non-eluting feature and a high corrosion resistance, such as titanium or a titanium alloy, an electrode wire is formed out of titanium or a titanium alloy or platinum or a platinum alloy so as not to spoil the inherently high non-eluting feature and corrosion resistance of these metal materials, and so as to enable a medical instrument to he produced as designed with a high machining precision. The manufacturing of a medical instrument of a complicated shape out of a metal material having high non-eluting feature and corrosion resistance is done by electric discharge machining so as not to spoil the inherently high non-eluting feature and corrosion resistance

and so as to prevent impurities from being mixed in the product from a machining jig, ensure the high corrosion resistance and non-eluting feature of the product in a living body, and contribute much to various kinds of industrial fields, such as medical, dental and stock farming fields.

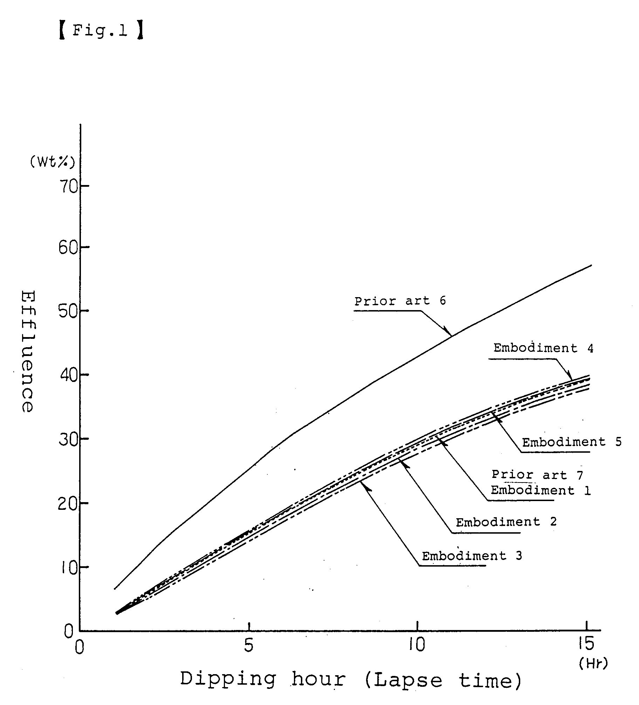

【Fig.1】

(0001)

FIELD OF THE INDUSTRIAL UTILIZATION

The disclosed technique belongs to the art of processing metal materials used as materials of such medical tools as commonly termed implant materials to be buried in the living body for medical purposes or surgical operation tools.

(0002)

PRIOR ART

As is well known in the art, the improvement of the civic file is supported by the prosperity of various chemical techniques. Aside form the convenience in daily life, it pronouncedly owes lifetime improvement owing to the process of medical therapy techniques as well as hygienics.

(0003)

Although drug administration and health control are providing great contributions, the striking process in the surgical therapy such as surgical operations is also greatly contributing.

(0004)

Further, it can not be missed that the remarkable development of various medical therapy tools including surgical operation tools are greatly contributing.

(0005)

Naturally, in order to be able to make full use of the intrinsic functions of the medical tools such as surgical operation tools, processing reliably meeting basic designs is very important. However, such chemical properties as freedom from effluence or corrosion resistance and such biological properties as freedom from attachment of bacteria should also be pursuited.

(0006)

Recently, owing to improvements of biological and anatomical techniques commonly called implant materials, which are used inside the living body such as being buried in the man's body, have been developed, and those those contributing more and more to the medical technique improvement have appeared.

(0007)

Metal materials which are used for medical tools, such as the implant materials and surgical instruments, are usually referred to as medical metal materials. In their use, their unchangeable fitness is required stringently such that they can maintain the freedom from effluence and sufficient corrosion resistance with respect to the biochemical atmosphere and environments in the living body and do not provide harmful activities in use.

(0008)

Historically, extensive biological tests, animal experiments and prudent clinical tests were conducted repeatedly to find out metal materials which can sufficiently meet the above stringent requirements, and now it is thought that titanium and alloys thereof (hereinafter collectively referred to as titanium alloys) are best.

(0009)

With the medical metal materials for implant materials, to be able to make full use of their functions, not only the design should be highly accurate, but also highly accurate processing after the design is very important as noted above. To meet these requirements, precision casting and precision machining are adopted for manufacture.

(0010)

While titanium metals are presently used as medical metal materials, precision casting and precision machining naturally are also required for the processing of such titanium metal materials.

(0011)

From the metal material standpoint and also from the metallurgical standpoint, the titanium metals, although excellent as the medical metal material so long as the freedom from effluence with respect to the living body and high corrosion resistance are concerned, are subject to difficulties in connection with the casting and machining properties.

(0012)

When they are to be processed by precision casting, they are subject to undesired casting defects such as cavity formation, resulting in insufficient mechanical strength or inferior corrosion resistance. Further, there are restrictions imposed on the precision machining.

(0013)

Therefore, they do not yet sufficiently meet the accuracy required for the medical tools, and in actual processing impurities are detected, which are introduced in various tools.

(0014)

In many cases of the processing of various precision machine devices and tools, unexpectedly complicated shapes are involved, causing difficulties in the processing. Further, there are increasing demands for increased accuracy for materials difficult to process. Among such circumstances, in various machine production industries extensive use is made of high precision processing techniques and discharge processing techniques, and continued researches and developments are being made. Discharge processing machines which are actually used are generally classified into two kinds. In one type, a thin electrode wire is used, which is moved in the longitudinal direction with a predetermined tension applied to it, and discharge is generated between the work and the electrode wire via intervening water or like processing liquid for predetermined processing of the work with the discharge energy thus generated. The other type of machine is called diesinking discharge processing machine, which is based on the same processing principles as the first-mentioned type of machine.

(0015)

For the electrode wire used for such discharge machine, copper or an alloy thereof is usually used from the standpoints of the electric conductivity, mechanical strength, durability, etc.

(0016)

While the discharge processing machine has a merit that it permits high accuracy processing as designed or nearly as designed, it has been experimentally known that part of the electrode wire material used has a character of being attached to the work.

4

(0017)

It has also been experimentally known that this fact is of course the case as well with copper or alloys thereof as the electrode wire material to be processed and also that in the case of works of titanium materials, which are recently regarded to be important as medical metal materials, the electrode wire material is attached to the titanium metal surface of the work. Therefore, the titanium metals, although having satisfactory fitness to the living body and excellent properties as the medical metal material, do not permit required design accuracy of processing to be obtained even with precision machine processing with a consequence that it is impossible to obtain their intrinsic functions as the medical metal material.

(0018)

Further, they do not permit processing of medical metal materials even by using the discharge processing technique, which permits highly accurate processing of highly difficultly processible works having complicated processing shapes.

(0019)

OBJECT OF THE INVENTION

An object of the invention of this application is to provide an excellent method of processing medical metal materials, which can solve the technical problems in the properties of the discharge processing in the case of using the titanium metals based on the above prior art techniques as medical metal materials, and permits even medical metal materials of work metals for processing with electrode wire to be processed as desired as medical metal materials of titanium metals by providing the freedom from effluence with respect to the living body and high corrosion resistance obtained on the basis of the repetition of experiments of application of process techniques to high accuracy, high corrosion resistance medical metal materials with theoretical analysis, experiment data collection and analytic study of heretofore accumulated peripheral techniques to the discharge processing technology.

(0020)

BACKGROUND OF THE INVENTION

The inventor has conducted logical and experimental versatility researches and investigations concerning the discharge processing techniques in alloy processing and peripheral techniques thereto, thus mastering and obtaining the way of realizing a technique, which can make titanium or alloys thereof as the medical metal material to be free from effluence in the living body, be highly corrosion-resistant and be sufficiently capable of highly accurate processing by discharge processing by using titanium or alloys thereof or platinum or alloys thereof prepared with predetermined addition of platinum family elements.

(0021)

MEANS AND FUNCTIONS FOR SOLVING THE PROBLEMS

To solve the above problems, the constitution or subject matter of the invention of the present application to meet the above object, as set forth in the previous claims, resides in technical means, in which, in carrying out accurate processing as discharge processing after design on titanium or an alloy thereof as the medical metal material for a medical tool such as an implant material or a surgical operation tool to be provided in the living body such as man's body, a titanium metal or platinum or a platinum metal prepared with addition of a platinum family element is used for the electrode wire for dischage processing more similar to the general status, thus permitting making full use of excellent intrinsic properties of the medical metal material, eliminating introduction of impurities from the processing tools and harmful activities, and permitting the provision of excellent medical tools as desired.

(0022)

EXAMPLES

Examples of the invention of the application will now be given together with prior art examples with reference to the drawing.

(0023)

Table 1 lists the examples as experiment examples together with the prior art examples.

(0024)

TABLE 1

As the discharge processing machine may be used a usual wire discharge processing machine. The material of the electrode wire used according to the invention of the application may be be of JIS Type 1, JIS Type 2 or JIS Type 3, or may be titanium alloys of JIS Type 12.

(0025)

It has been confirmed that platinum or an alloy thereof prepared by adding a predetermined amount of any of platinum family elements (referred to as platinum metal), which, like titanium, is among a few metals confirmed by biological experiments and animal experiments to be free from effluence in a living body it is buried in, be excellently corrosion-resistant and has no harmful activity on the living body, may be used without spoiling the excellent freedom from effluence and high corrosion resistance of titanium or an alloy thereof (referred to as titanium metal) as the medical metal material.

(0026)

The electrode wire noted above was prepared by well-known melting processing ways.

(0027)

Specifically, titanium rods conforming to JIS Type 2 titanium and JIS Type 12 titanium alloys were heat treated and elongated. As for the platinum metal, it is melted by arc melting, which is again a well-known melting technique, followed by heat treatment and elongation. In these ways, wires with a diameter of 0.2 mm and a length of 3,000 m and having a predetermined shape were produced.

(0028)

As for the platinum family element to be added for the platinum metal, it has been experimentally confirmed that palladium, iridium, rhodium and lutetium are satisfactory from the standpoints of the mechanical properties and corrosion resistance of the wire material of the electrode wire and that from the standpoint of the processibility they are ideally added by 30 % or below.

(0029)

As the titanium metal, JIS Typ2 2 metal materials and JIS Type 2 titanium alloys were used, which are presently thought to be best from the biological tests, animal experiments and clinical tests.

(0030)

As for wire discharge processing conditions, the pulse duration was set to 0.15 to 85 $\mu$sec., the pulse pause time to 2 to 16 $\mu$sec., the current peak to 4.5 to 58 A, the non-load voltage to 30 to 100 V, the electrode wire feed speed to 6 to 15 m/min., the electrode wire tension to 0.1 to 2.0 kgf, and the processing speed to 0.1 to 2 mm/min. As a result, it was confirmed that specific processing conditions permit non-processed medical metal materials to be processed as desirned with high accuracy.

(0031)

Now, examples among the experiment examples shown in Table 1 noted above will be described. In the experiment data of Experiment Example 1, pure titanium of JIS Type 2 was used as the medical metal material, and pure titanium JIS Type 2 as the electrode wire material. The pulse duration was set to 0.2 $\mu$sec., the pulse pause time to 12.0 $\mu$sec., the current peak to 29.0 A, the non-load voltage to 100 V, the electrode wire feed speed to 7.0 m/min., the electrode wire tension to 1.0 kgf, and the processing speed to 0.5 mm/min. In the experiment data of Experiment Example 4, as the medical metal material was used JIS Type 2 titanium alloy material. As the electrode wire material was used copper for first processing, and pure titanium material of JIS Type 2 for second processing. In the second processing, the surface in the first processing was discharge processed with 100 $\mu$m. As for the discharge processing conditions for the first processing, the pulse duration was set to 0.5 $\mu$sec., the pulse pause time to 16.0 $\mu$sec., the current peak to 18.0 A, the non-load voltage to 70 V, the electrode wire feed speed to 7.0 m/min., the electrode wire tension to 1.0 kgf, and the processing speed to 2.0 mm/min. As for the discharge processing conditions for the second processing, the pulse duration was set to 0.2 $\mu$sec., the pulse pause time to 12.0 $\mu$sec., the curernt peak to 29.0 A, the non-load voltage to 90.0 V, the electrode wire speed to 7.0 m/min., the electrode wire tension to 10.0 kgf, and the processing speed to 1.5 mm/min.

(0032)

The data in this Experiment Example 4 were obtained from study experiments conducted by the inventor in various aspects, and they indicate more economical processing conditions for the discharge processing speed with respect to the titanium metal as the medical metal material can be reduced.

(0033)

Fig. 1 shows results of measurements of the titanium effluence with the lapse of time, conducted on titanium metal test piece as the medical metal material prepared in the above way, by dipping the test piece after alcohol washing thereof in 35 % hydrochloric acid as a cruel environment corrosion-resistant test condition.

(0034)

As is seen from Fig. 1, compared to the prior art status of use of the copper electrode wire as well-known electrode wire, with the medical metal material processed according to the invention of the present application the titanium effluence is very small, thus indicating this medical metal material to be excellent even in comparison with the well-known titanium material.

(0035)

EFFECTS OF THE INVENTION

As has been shown in the foregoing, the basically processed medical metal material of titanium metal according to the invention does not only permit high accuracy of processing capable of permitting making full use of the intrinsic functions of the medical metal material, but also is free from intrusion of impurities from the processing tool, free from spoiling the excellent corrosion resistance of titanium metal and thus sufficiently free from effluence in the living body and highly corrosion-resistant, thus permitting an excellent medical metal material free from harmful activities to be obtained.

(0036)

Further, it is possible to make full use of the properties of the precision processing by discharge processing for such medical therapy tools as implant materials and surgical operation tools requiring stringent high accuracy processing. Further, accurate processing as designed can be obtained irrespective of processing difficulties due to complicated shapes and also due to materials. Thus, great contribution can be given to the fields of medical, dental and so forth therapy. Further, great contribution can be given to the industries of manufacture of various machines and discharge processing.

(0037)

Thus, it is possible to obtain ultimate effects of improving the average life of the mankind and the quality of hygenics and also greatly contribute to livestock industries. This merit is worth over-evaluation in the industries.

BRIEF DESCRIPTION OF THE DRAWING

FIGURE 1

A graph showing titanium effluence measurement data in experiment examples according to the invention of the present application and prior art examples.

TABLE 1

| Experiment Example No. | examples according to the invention | | | | | prior art examples | |
|---|---|---|---|---|---|---|---|
| | 1 | 2 | 3 | 4 | 5 | 6 | 7 |
| Medical metal material | Pure titanium JIS Type 2 | Pure titanium JIS Type 2 | Pure titanium JIS Type 2 | Pure titanium JIS Type 12 | Titanium alloy | Pure titanium JIS Type 2 | Pure titanium JIS Type 2 |
| Electrode wire material | Pure titanium JIS Type 2 | Pt | 90 % Pt 10 % Ir | first processing: Cu / second processing: Pure titanium JIS Type 2 | Titanium alloy JIS Type 12 | Cu | Material obtained by polishing work material with water resistant polishing paper # 1000 |
| **Wire discharge processing conditions** — Pulse duration (μs) | 0.2 | 0.3 | 0.3 | 0.5 | 0.2 | 0.3 | 0.5 |
| Pulse pause time (μs) | 12.0 | 14.0 | 15.0 | 16.0 | 12.0 | 13.0 | 16.0 |
| Current peak (A) | 29.0 | 21.0 | 23.0 | 18.0 | 29.0 | 28.0 | 18.0 |
| Non-load voltage (V) | 100.0 | 65.0 | 60.0 | 70.0 | 90.0 | 80.0 | 70.0 |
| Wire feed speed (m/min.) | 7.0 | 6.0 | 7.5 | 7.0 | 7.0 | 6.5 | 7.0 |
| Wire tension (kgf) | 1.0 | 0.4 | 0.4 | 1.0 | 1.0 | 1.0 | 1.0 |
| Processing speed (mm/min.) | 0.5 | 1.0 | 0.8 | 2.0 | 1.8 | 0.8 | 2.0 |

All the samples after the discharge processing have a shape with a thickness of 1.00 mm, a width of 10.00 mm and a length of 10.00 mm.

**Claims**

1. A method of processing medical metal materials, in which a titanium metal matrerial for a medical metal product made of a titanium metal is processed by discharge processing, characterized in that an electrode wire used for said discharge processing is made of a titanium metal or a platinum metal.

2. The method of processing medical metal materials according to claim 1, characterized in that said titanium metal is titanium or a titanium alloy.

3. The method of processing medical metal material according to claim 1, characterized in that said platinum metal is platinum or a platinum-family-added platinum metal.

【Fig.1】

Prior art 6

Embodiment 4

Embodiment 5

Prior art 7
Embodiment 1

Embodiment 2

Embodiment 3

(Wt%)

Effluence

Dipping hour (Lapse time)

(Hr)

| | International application No. |
|---|---|
| | PCT/JP93/00169 |

**A. CLASSIFICATION OF SUBJECT MATTER**

Int. Cl$^5$  B23H7/08

According to International Patent Classification (IPC) or to both national classification and IPC

**B. FIELDS SEARCHED**

Minimum documentation searched (classification system followed by classification symbols)

Int. Cl$^5$  B23H7/08

Documentation searched other than minimum documentation to the extent that such documents are included in the fields searched

| Jitsuyo Shinan Koho | 1926 - 1993 |
|---|---|
| Kokai Jitsuyo Shinan Koho | 1971 - 1993 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP, A, 63-203262 (Hitachi Metals, Ltd.), August 23, 1988 (23. 08. 88), Lines 3 to 7, lower left part of column 2 (Family: none) | 1-3 |
| Y | JP, B2, 63-48656 (Mitsubishi Shinko K.K., Daiichi Denko K.K., Mitsubishi Electric Corp.), September 30, 1988 (30. 09. 88), Lines 7 to 10, column 6, Fig. 2 (Family: none) | 1, 2 |
| Y | JP, A, 63-81018 (Toray Industries, Inc.), April 11, 1988 (11. 04. 88), Lines 17 to 20, lower right part of column 2, table 3 (Family: none) | 1, 3 |

☒ Further documents are listed in the continuation of Box C.　　☐ See patent family annex.

| | |
|---|---|
| * Special categories of cited documents:<br>"A" document defining the general state of the art which is not considered to be of particular relevance<br>"E" earlier document but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| April 13, 1993 (13. 04. 93) | May 11, 1993 (11. 05. 93) |

| Name and mailing address of the ISA/ | Authorized officer |
|---|---|
| Japanese Patent Office | |
| Facsimile No. | Telephone No. |

Form PCT/ISA/210 (second sheet) (July 1992)